(19) Europäisches Patentamt
European Patent Office
Office européen des brevets

(11) Veröffentlichungsnummer: **0 649 649 A2**

# (12) EUROPÄISCHE PATENTANMELDUNG

(21) Anmeldenummer: **94116068.1**

(51) Int. Cl.[6]: **A61K 9/10**, A01N 25/04

(22) Anmeldetag: **12.10.94**

(30) Priorität: **25.10.93 DE 4336309**
**30.06.94 DE 4422881**

(43) Veröffentlichungstag der Anmeldung:
**26.04.95 Patentblatt 95/17**

(84) Benannte Vertragsstaaten:
**AT BE CH DE DK ES FR GB IT LI NL PT SE**

(71) Anmelder: **BAYER AG**

**D-51368 Leverkusen (DE)**

(72) Erfinder: **Goossens, John, Ir.**
**Hebbelstrasse 5**
**D-51373 Leverkusen (DE)**
Erfinder: **Penners, Günther, Dr.**
**Körnerstrasse 6**
**D-51373 Leverkusen (DE)**
Erfinder: **Dutzmann, Stefan, Dr.**
**Kosenberg 10**
**D-40721 Hilden (DE)**
Erfinder: **Dehne, Heinz-Wilhelm, Prof. Dr.**
**Charles-Wimar-Strasse 15**
**D-53125 Bonn (DE)**
Erfinder: **Brüggen, Kai-Uwe, Dr.**
**Hebbelweg 41**
**D-45549 Sprockhövel (DE)**

(54) **Kolloidal dispergierbare Wirkstoff-Formulierungen.**

(57) Die vorliegende Anmeldung betrifft den Einsatz von Copolymeren auf der Basis von Vinylpyrrolidon bei der Formulierung von schwer wasserlöslichen und gegebenenfalls schwer in apolaren Lösungsmittel löslichen Wirkstoffen.

EP 0 649 649 A2

Die vorliegende Erfindung betrifft den Einsatz von Copolymeren auf der Basis von Vinylpyrrolidon bei der Formulierung von schwer wasserlöslichen und gegebenenfalls schwer in apolaren Lösungsmittel löslichen Wirkstoffen.

Die Bioverfügbarkeit und somit die Wirkung eines Wirkstoffes, wird stark durch seine Löslichkeit bestimmt. In der Regel kann ein Wirkstoff nur in gelöster Form seine physiologische Wirkung entfalten. Deshalb treten bei schwer löslichen Wirkstoffen oft Schwierigkeiten bei der Bereitstellung wirksamer Mittel zur Verabreichung oder Ausbringung auf.

Durch unterschiedliche Formulierungsoptionen, die entweder die Wasserlöslichkeit des Wirkstoffes, oder aber die Lösegeschwindigkeit verbessern, versucht man die Bioverfügbarkeit schwer löslicher Wirkstoffe zu erhöhen und damit die notwendige Dosis zu erniedrigen.

So wird in US-4 412 986 durch die Herstellung amorpher Wirkstoff-Copräzipitate mit wasserkompatiblen Polymeren wie Polyvinylpyrrolidon (PVP) die Löslichkeit der Wirksubstanz wesentlich erhöht, weil der Wirkstoff nach Lösung des Copräzipitates über längere Zeit in übersättigter Form vorliegt. PVP, eventuell in Kombination mit anderen Hilfsstoffen wie z.B. Tenside wird vielfältig bei der Formulierung kristallisationsstabiler wäßriger Wirkstoff-Formulierungen eingesetzt. So beschreiben EP 212 853 A2, JP 04 018 015, JP 04 018 022, JP 04 149 132, JP 03 287 535 und JP 02 264 716 die Bereitstellung von PVP-haltigen Phenytoin- bzw. Clemastin-Fumarat, Piroxicam, Danazol, Pranoprofen bzw. Tranilast-Lösungen.

Als Alternative wird die Herstellung kolloidaler Dispersionen mittels Fällung aus organischen Wirkstofflösungen beschrieben. Durch Fällung lassen sich im Prinzip kolloidale wäßrige Dispersionen, auch Hydrosole genannt, erzeugen, deren Teilchengröße bei <0,1 $\mu$ liegt. So beschreiben z.B. M. List, in seiner 1987 an der Universität Basel unter dem Titel "Hydrosole, eine intravenöse Arzneiform zur Herstellung von Injektionen und Infusionen in Wasser schwer löslicher Wirkstoffe" erschienenen Inauguraldissertation und DE 3 742 473 die Herstellung kolloidaler, intravenös applizierbarer Dispersionen. Die Herstellung erfolgt durch das Vermischen einer Lösung eines im Wasser schwerlöslichen Wirkstoffes in einem mit Wasser mischbaren organischen Lösemittel mit einer wäßrigen Vorlage, wobei sowohl die organische als auch die wäßrige Phase Stabilisatoren bzw. andere Additive enthalten kann. US 4 826 689 beschreibt die Herstellung monodisperser kolloidaler Dispersionen durch die dosierte Zugabe einer wäßrigen Stabilisatorlösung zu einer Lösung des Wirkstoffes in einer reinen oder gegebenenfalls Cosolvent enthaltenden organischen Phase, wobei die Herstellungsbedingungen wie Ansatzvolumen, Temperatur, Rühr- und Dosiergeschwindigkeit stark die Qualität der Dispersion bestimmten. Nach DD 293 727 A lassen sich kolloidale Dispersionen des Wirkstoffes 2-Hydroxy-5-methyl-laurophenon mittels Verwendung von PVP als Stabilisator herstellen.

Die genannten, dem Stand der Technik entsprechenden Formulierungsprinzipien sind jedoch mit einigen Nachteilen behaftet. Copräzipitate ergeben zwar nach Auflösung in Wasser eine übersättigte Wirkstofflösung, der Ubersättigungsgrad ist jedoch in den meisten Fällen relativ gering. Außerdem kristallisieren die übersättigten Lösungen im Laufe der Zeit wieder aus, was sich negativ auf die Bioverfügbarkeit des Wirkstoffes auswirkt. Zudem wird die Applikation des Wirkstoffes erschwert. So sind z.B. in Spritzgeräten, die üblicherweise zur Ausbringung wäßriger Formulierungen von Pflanzenschutzmitteln verwendet werden, mehrere Filter sowie Düsen vorhanden. Alle diese Filter sowie auch die Düsen können bei der Ausbringung von wäßrigen Spritzbrühen auf Basis fester Wirkstoffe mehr oder weniger leicht durch auskristallisierenden Wirkstoff verstopfen.

Kritisch bei der Herstellung kolloidaler Dispersionen aus Wirkstoffen mit starker Kristallisationstendenz durch Fällung aus organischer Lösung ist die Konversion der direkt nach der Fällung amorph vorliegenden Teilchen in die thermodynamisch stabilere kristalline Phase. In US 4 826 689 wird durch Kontrolle der Fällbedingungen der Wirkstoff über einige Zeit in diesem amorphen, metastabilen Zustand stabilisiert. Jedoch wird in dieser Patentschrift auf die Notwendigkeit der Abtrennung des Fällproduktes aus dem Dispersionsmedium und nachträglicher Trocknung zur Vermeidung von Rekristallisation der amorphen Wirkstoffteilchen hingewiesen. Zur Herstellung eines mit Hinblick auf die Teilchengröße stabilen Hydrosols muß das Lösemittel nach der Fällung entfernt werden. Aus der 1990 an der Universität Basel erschienenen Inauguraldissertation von P. Gaßmann mit dem Titel "Herstellung und Stabilisierung von Hydrosolen zur Intravenösen Applikation" geht hervor, daß bei der Herstellung von Hydrosolen aus Wirkstoffen aus ein und der selben Substakzklasse die Stabilität stark von den strukturellen und physikochemischen Merkmalen der einzelnen Substanzen abhängt. So wiesen Versuche mit den Dihydropyridinen Darodipin und Isradipin aus, daß Hydrosole aus Isradipin bis zu 20 mal stabiler sind als vergleichbare Hydrosole.

Aus dem Stand der Technik geht hervor, daß die Bereitstellung von Formulierungen schwer löslicher Wirkstoffe, die sich durch gute Wirksamkeit und gutes Applikationsverhalten auszeichnen auf der Basis der verfügbaren Formulierungsprinzipien, und dabei verwendeten Formulierungshilfsstoffen nur schwer zu erreichen ist. Eine formulierungsbedingte Erhöhung der Wirksamkeit ermöglicht jedoch eine Reduzierung der notwendigen Wirkstoffdosis was wiederum zu geringeren Nebenwirkungen und Umweltbelastungen

führt.

Gegenstand der Erfindung ist die Verwendung von hydrophobe Comonomere enthaltenden Vinylpyrrolidon und/oder Vinylcaprolactam Copolymerisaten in Formulierungen von schwer wasserlöslichen Wirkstoffen.

Die Formulierungen bilden in wäßrigen Medien homogene Lösungen oder kollodiale Dispersionen, die sich im Vergleich zu gängigen wäßrigen Wirkstoff-Formulierungen durch eine deutlich erhöhte Stabilität, bessere Applikationseigenschaflen und durch erhöhte Wirksamkeit auszeichnen.

Im wesentlichen enthalten die erfindungsgemäßen Formulierungen neben dem Wirkstoff (A) ein Vinylpyrrolidon- und/oder Vinylcaprolactam Vinylester-Copolymerisat (B), ein Tensid (C) und gegebenenfalls Additive (D) und/oder ein Lösemittel (E).

Copolymerisate (B) im Sinne der Erfindung sind statistische Copolymerisate aus mindestens einem Vinylpyrrolidon und/oder Vinylcaprolactam und hydrophoben Vinylmonomeren. Beispiele dieser Vinylmonomeren sind höhercyclische Vinyllactame, kernalkyliertes Vinylpyrrolidon, Vinylcaprolactam, Vinylester, Vinylether, Styrol, Maleinsäureanhydrid und Mono- oder Diester der Maleinsäure mit linearen oder verzweigten Alkoholen. Besonders bevorzugt sind statistische Copolymerisate von Vinylpyrrolidon und Vinylester der Integral-Formel

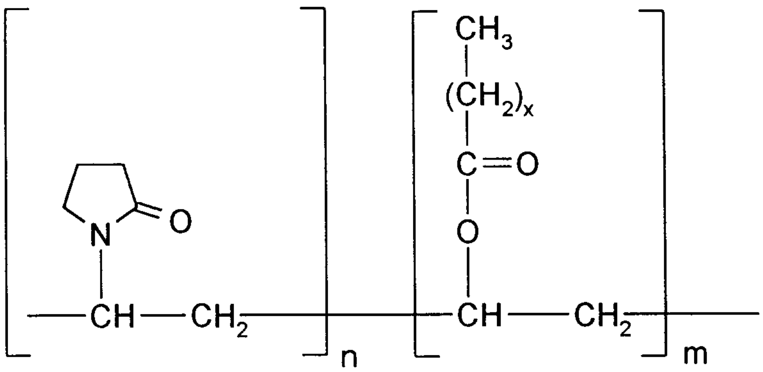 ,

in welcher x zwischen 0 und 16, vorzugsweise zwischen 0 und 6 liegt, und n und m größer 1, vorzugsweise größer 10 sind und der Gewichtsanteil des Vinylesters am Gesamtpolymer 5-90 %, vorzugsweise zwischen 10 und 80 % und besonders bevorzugt zwischen 30 und 80 % beträgt.

Copolymerisate aus Vinylpyrrolidon und Vinylacetat (x = 0) sind z. B. unter dem Handelsnamen Luviskol VA® bei der Firma BASF bzw. unter dem Handelsnamen Agrimer VA® bei der Firma ISP erhältlich.

Diese Copolymere auf der Basis von Vinylpyrrolidon sind selbst in Wasser nur schlecht, bzw. gar nicht löslich. Daher ist es um so überraschender das diese Copolymere bei der Herstellung wäßriger Wirkstoffdispersionen und -Lösungen verwendet werden können, und dabei eine wesentlich bessere Stabilität erzeugen als bekannte Standardformulierungen, die als Stabilisator z.B. ein reines wasserlösliches Polyvinylpyrrolidon oder nur Tenside enthalten.

Unter den Tensiden (C) im Sinne der Erfindung kommen nichtionogene, kationische und anionische Emulgatoren, wie Polyoxyethylen-Fettsäure-Ester, Polyoxyethylen-Feffalkohol-Ether, Alkylaryl-Polyglykol-Ether, Alkylsulfonate, Alkylsulfate, Alkylarylsulfonate und Arylsulfate in Betracht. Beispiele solcher Tenside sind im Lexikon der Hilfsstoffe (Fiedler, H.P. "Lexikon der Hilfsstoffe für Pharmazie, Kosmetik und angrenzende Gebiete", Edition, Cantor Aulendorf , (1981)) aufgeführt. Sie können einzeln oder auch in Mischung eingesetzt werden. Besonders geeignete Tenside im Sinne der Erfindung sind ionische Tenside wie Na-Dodecylsulfat und Na-Dioctylsulfosuccinat und Dodecylbenzolsulfonsäure-Monoethanolaminsalz.

Additive (D) in den erfindungsgemäßen Formulierungen, sofern es sich hierbei um Formulierungen für Pflanzenschutzwirkstoffe handelt, sind Kältestabilisatoren, die die Lagereigenschaften der Formulierung bei niedriger Temperatur verbessern und Haftmittel, die sich positiv auf die Adhäsion der aus der Formulierungen hergestellten wäßrigen Spritzbrühe an der Pflanze auswirken. Beispiele der Kältestabilisatoren sind Harnstoff, Glycerin und Propylenglykol. Als Haftmittel kommen vorzugsweise in Betracht Carboxymethylcellulose, natürliche und synthetische Polymere, wie Gummi arabicum, Polyvinylalkohol, Polyvinylacetat sowie natürliche Phospholipide, wie Kephaline und Lecithine, und auch synthetische Phospholipide.

Als Lösemittel (E) können alle üblicherweise für derartige Zwecke einsetzbaren polaren und unpolaren organischen Solventien verwendet werden. In Frage kommen Propylenglykol, Polyethylenglykol mit unterschiedlichem Molekulargewicht, Ketone, wie Methyl-isobutylketon und Cyclohexanon, ferner Amide, wie Dimethylformamid, weiterhin cyclische Verbindungen, wie N-Methyl-pyrrolidon, N-Methylcaprolactam und Butyrolacton, darüber hinaus stark polare Solventien, wie Dimethylsulfoxid, ferner aromatische Kohlenwasserstoffe, wie Xylol, außerdem Ester, wie Propylglykol-monomethylether-acetat, Adipinsäure-dibutylester, Essigsäurehexylester, Essigsäureheptylester, Zitronensäure-tri-n-butylester und Phthalsäure-di-n-butylester, Glykolether wie Propylenglykolmethylether und weiterhin Alkohole, wie Ethanol, n- und i-Propanol, n- und i-Butanol, n- und i-Amylalkohol, Benzylalkohol, Tetrahydrofurylalkohol und 1-Methoxy-2-propanol.

Besonders bevorzugt sind N-Methylpyrrolidon, Dimethylformamid, Dimethylsulfoxid, Propylenglykol, Polyethylenglykol, Ethanol und i-Propanol.

Prinzipiell können die erfindungsgemäßen Formulierungen für alle Wirkstoffe verwendet werden. Vorzugsweise sind die Formulierungen jedoch geeignet für Wirkstoffe (A), die bei Raumtemperatur eine Wasserlöslichkeit von <0,5 g/100 ml, speziell <0,1 g/100 ml und besonders <0,01/100 ml, aufweisen. Beispiele solcher Wirkstoffe sind die bekannten Dihydropyridine wie beispielsweise Nimodipin, Nifedipin, Nitrendipin, Nisoldipin, Darodipin, Isradipin, Felodipin oder

**Fungizide:**

2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)o-tolyl] acetat]; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazal, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurance, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Tebuconazol, Tecloftalam, Tecnazen, Tetraconazol, Thiabendazol, Thicycofen, Thiophanat-methyl, Thiram, Tolclophos-methyl, Tolylfluanid, Triadimefon, Triadimenol, Triazoxid, Trichlamid, Tricycloazol, Tridemorph, Triflumizol, Triforin, Triticonazol,
Validamycin A, Vinclozolin,
Zineb, Ziram

**Insektizide / Akarizide / Nematizide:**

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chlorethocarb, Chlorethoxyfos, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotohphos, Diethion, Diflubenzuron, Dimethoat, Dimethylvinphos, Dioxathion, Disulfoton, Edifenphos, Emamectin, Esfenvalerat, Ethiofencarb, Ethion, Ethofenprox, Ethoprophos, Etrimphos, Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox, Imidacloprid, Iprobenfos, Isazophos, Isofenphos, Isoprocarb, Isoxathion, Ivemectin, Lamda-cyhalothrin, Lufenuron. Malathion, Mecarbam, Mervinphos, Mesulfenphos, Metaldehyd, Methacrifos, Methamidophos, Methidiathion, Methiocarb, Methomyl, Metolcarb, Milbemectin, Monocrotophos, Moxidectin, Naled, NC 184, Nitenpyram, Omethoat, Oxamyl, Oxydemethon M, Oxydeprofos, Parathion A, Parathion M, Permethrin, Phentoat, Phorat, Phosalon, Phosmet, Phosphamdon, Phoxim, Pirimicarb, Pirimiphos M, Pirimiphos A, Profenofos, Profenophos, Promecarb, Propaphos, Propoxur, Prothiofos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen, Quinalphos, Salithion, Sebufos, Silafluofen, Sulfotep, Sulprofos, Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb, Vamidothion, XMC, Xylylcarb, Zetamethrin.

**Herbizide**

Anilide, wie z.B. Diflufenican und Propanil; Acrylcarbonsäure, wie z.B. Dichlorpicolinsäure, Dicamba und Picoloram; Aryloxyalkansäuren, wie z.B. Fluroxypyr und Triclopyr, Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate` wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Formesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Alnidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfüron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Die Wirkstoffe können einzeln, oder in Kombination mit anderen löslichen oder unlöslichen Wirkstoffen in der Formulierung enthalten sein.

Die erfindungsgemäßen Formulierungen werden durch einfaches Vermischen, gegebenenfalls unter Erwärmung, hergestellt.

Die erfindungsgemäßen Formulierungen können als solche in Form von Konzentrate oder in Form von Pflanzenschutzmitteln oder Arzneimitteln für den oralen, parenteralen und transdermalen Einsatz verwendet werden. Im Pflanzenschutz können sie sowohl für die Blatt-, Boden- und Stammapplikation als auch bei der Saatgutbehandlung verwendet werden.

Durch Eindispergieren der erfindungsgemäßen Formulierungen in eine wäßrige Phase bilden sich, je nach Art des eingesetzten Polymeren (B) und des verwendeten Tensides (C), homogene Lösungen oder kolloidiale Dispersionen, die sich durch hohe Stabilität auszeichnen. Diese wäßrigen Systeme stellen die Applikationsform der Formulierungen dar. Die Wirkstoffkonzentration in der wäßrigen Applikationsform beträgt zwischen 0,0001 und 3 Gew.-%, vorzugsweise zwischen 0,001 und 2 Gew.-%. Zur Herstellung

solcher wäßrigen Lösung bzw. Dispersion wird die erfindungsgemäße Formulierung gegebenenfalls unter Rühren und/oder Pumpen mit der jeweils gewünschten Menge an wäßriger Phase vermischt. Die Verwendung von speziellen Dispergieraggregaten wie Scherhomogenisatoren ist dabei nicht erforderlich.

Das Verhältnis von Wirkstoff (A) zu Polymer (B) kann innerhalb eines weiten Bereiches variiert werden und ist durch Vorversuche leicht bestimmbar. Im allgemeinen liegt das Gewichtsverhältnis von Wirkstoff zu Polymer je nach Wirkstoff zwischen 1:0,2 und 1:10, vorzugsweise zwischen 1:0,2 und 1:3. Die Mengen an weiteren Zusatzstoffen in der erfindungsgemäßen Formulierung können innerhalb eines größeren Bereiches variiert werden. Sie sind jedoch so eingestellt, daß die Formulierung nach einfacher Vermischung mit Wasser eine homogene Lösung oder aber eine kolloidiale Dispersion mit Teilchen im Größenbereich 0,01-0,4 µm, vorzugsweise im Größenbereich 0,03-0,2 µm bildet.

Die folgenden Beispiele dienen zur Erläuterung der Erfindung.

**Beispiel 1**

1 Gew.-Teil Nimodipin, 5 Gew.-Teile eines Copolymerisates aus 30 Gew.-% Vinylpyrrolidon und 70 Gew.-% Vinylacetat und 0,225 Gew.-Teile Natriumdodecylsulfat werden in 19 Gew.-Teile Ethanol (96 %) gelöst. Dieses Konzentrat wird in 225 Gew.-Teile Wasser eindispergiert. Bei der Dispergierung wird die Wasserphase vorgelegt, und das Konzentrat unter Rühren zügig zugegeben. Obwohl sowohl der Wirkstoff als auch das eingesetzte Polymer in reiner Form wasserunlöslich sind, entsteht eine klare Lösung, die auch nach längerer Standzeit nicht eintrübt.

Als Maß für die Kristallisationstendenz des Wirkstoffes in der wäßrigen Dispersion wird der Gehalt an gelöstem Wirkstoff nach einer definierten Standzeit von 24 Stunden bei Raumtemperatur bestimmt. Hierbei wird folgendermaßen vorgegangen: 24 Stunden nach Herstellung der Dispersion wird dem Ansatz eine Probe entnommen und zur Entfernung eventueller kristallisierter Wirkstoffanteile durch 0,45 µm-Filter (Marke Millipore) filtriert. Eine definierte Filtratmenge wird in Ethanol gelöst und der Wirkstoffgehalt spektrophotometrisch bestimmt. Durch den Bezug des experimentell bestimmten Wirkstoffgehalt auf den theoretischen Gehalt kann der Anteil an gelöstem und/oder kolloidal vorliegenden Wirkstoff ermittelt werden.

Der Gehalt an filtrierbarem Wirkstoff beträgt nach 24 Stunden 82 % der eingesetzten Konzentration von 4 mg/ml.

**Vergleichsbeispiel 1**

Gemäß Beispiel 1 wird ein wirkstoflhaltiges Konzentrat hergestellt, jedoch das Copolymerisat durch gleiche Gewichtsanteile eines reinen wasserlöslichen Polyvinylpyrrolidons (Luviskol K 30 der Firma BASF) ersetzt. Sofort nach der Herstellung trübt die wäßrige Phase, bedingt durch Wirkstoff-Kristallisation stark ein. Bereits nach einer Stunde bildet der kristallisierte Wirkstoff einen dicken Bodensatz und ist der Überstand klar und praktisch farblos, was auf eine quantitative Kristallisation des Wirkstoffes schließen läßt. Der Gehalt an filtrierbarem Wirkstoff 24 Stunden nach Herstellung beträgt 0 %.

**Beispiel 2**

1 Gew.-Teil Nimodipin, 4 Gew.-Teile eines Copolymerisates aus 20 Gew.-% Vinylpyrrolidon und 80 Gew.-% Vinylacetat und 0,225 Gew.-Teile Natriumdodecylsulfat werden in 15 Gew.-Teile Ethanol (96 %) gelöst. Dieses Konzentrat wird in 225 Gew.-Teile Wasser eindispergiert. Bei der Dispergierung wird die Wasserphase vorgelegt, und das Konzentrat unter Rühren zügig zugegeben. Es bildet sich eine kolloidiale opaleszente Dispersiom, die sich auch nach 24 stündiger Standzeit visuell nicht ändert. Der Gehalt an filtrierbarem Wirkstoff betrug nach 24 Stunden 87 % der eingesetzten Konzentration von 4 mg/ml.

**Beispiele 3 bis 7**

1 Gew.-Teil Nimodipin, 5 Gew.-Teile eines Copolymerisates hergestellt aus den in der Tabelle 1 angegebenen Gewichtsanteilen Vinylpyrrolidon und Vinylacetat sowie 0,0113 Gew.-Teile Natriumdodecylsulfat werden in 19 Gew.-Teile Ethanol (96 %) gelöst. Dieses Konzentrat wird gemäß Beispiel 1 in 225 Gew.-Teile Wasser eindispergiert. Es bilden sich, je nach Monomerverhältnis in dem eingesetzten Polymer homogene Lösungen oder kolloidiale opaleszente Dispersionen, die ihr Aussehen nach 24 Stunden nicht verändern, oder aber praktisch quantitativ auskristallisieren. Der in der Tabelle angegebene Gehalt an filtrierbaren Wirkstoff wurde nach einer Standzeit von 24 Stunden gemäß Beispiel 1 bestimmt. Es zeigt sich, daß mit zunehmenden Anteil an Vinylacetat die kristallisationsinhibierende Wirkung des Copolymerisats

zunimmt. Reines Vinylacetat ist jedoch zur Herstellung der erfindungsgemäßen Formulierungen nicht geeignet.

**Vergleichsbeispiel 2**

Die Dispersion des Vergleichsbeispieles 2 wurde im wesentlichen gemäß dem Beispiel 1 der DE 37 42 473 hergestellt, Darodipin wurde jedoch durch Nimodipin als Wirkstoff aus der Substanzklasse der Dihydropyridine ersetzt:

In 40 ml Ethanol 96 % werden 1 g Ethylcellulose N7 (DOW Chemical) und 0,4 g Nimodipin gelöst. Diese Lösung wird unter starkem Rühren schnell in 200 ml Aqua dest. 20 °C gegossen. Im Rotationsverdampfer bei 50 °C wird unter vermindertem Druck 5 min Ethanol abgedampft. Gemäß Beispiel 1 wird der Wirkstoffgehalt 24 Stunden nach Filtration durch 0,45 µm-Filter bestimmt. Der Wirkstoffgehalt beträgt 21 % des theroretischen Maximalgehaltes.

Tabelle 1

| Monomerverhältnis im Polymer (Gew.-%) | | | Aussehen der wäßrigen Mischung | | Wirkstoffgehalt nach 24 Std. |
|---|---|---|---|---|---|
| | | | nach Herstellung | nach 24 Std. | |
| 3. | Vinylpyrrolidon<br>Vinylacetat | 100<br>0 | klar | Kristalle in klarem Überstand | 3 |
| 4. | Vinylpyrrolidon<br>Vinylacetat | 60<br>40 | klar | Kristalle in klarem Überstand | 9 |
| 5. | Vinylpyrrolidon<br>Vinylacetat | 50<br>50 | klar | Kristalle in opaleszentem Überstand | 29 |
| 6. | Vinylpyrrolidon<br>Vinylacetat | 30<br>70 | opaleszent | opaleszent | 82 |
| 7. | Vinylpyrrolidon<br>Vinylacetat | 20<br>80 | opaleszent | opaleszent | 88 |
| 8. | Vinylpyrrolidon<br>Vinylacetat | 0<br>100 | grob partikulär | grob partikulär | 0 |

Zur Herstellung einer Prüflösung verdünnt man die zu prüfende Formulierung mit Wasser auf die gewünschte Konzentration. Die Spritzbrühen der nachfolgenden anwendungsgemäßer Formulierungen, sind alle transsudent. Läßt man das Polymer in den erfindungsgemäßen Formulierungen heraus oder versetzt dieses durch reines Polyvinylpyrrolidon so ergeben sich grob disperse, trübe und nicht stabile Spritzbrühen.

**Beispiel 9**

Der Wirkstoff [2-Methyl-1[[[1-(4-methylphenyl)ethyl]amino]carbonal]propyl]-carbaminsäure-1-methylester wird als erfindungsgemäßes organisches Konzentrat unter Verwendung der in der folgenden Tabelle mengenmäßig aufgeführten Bestandteile zubereitet. Nach Verdünnung des Konzentrates mit Wasser wird eine lichtdurchlässige wäßrige kolloidale Dispersion erhalten, die gegen Kristallisation und Sedimentieren beständig ist. Wird das Polymerisat Luviskol VA® weggelassen oder durch ein reines Polyvinylpyrrolidon wie Luvisol K 30® ersetzt, erhält man nach Verdünnung des Konzentrates mit Wasser trübe Dispersionen mit groben Teilchen, die sich innerhalb weniger Minuten absetzen. Zum Vergleich der biologischen Wirksamkeit wird eine Standardformulierung des Wirkstoffkonzentrates in Form einer Suspension wie in der Tabelle angegeben zubereitet.

| erfindungsgemäße Formulierung: | Standardformulierung: 25%iges netzfähiges Pulver |
|---|---|
| 15% Wirkstoff | 25% Wirkstoff |
| 5% Emulgator 1 | 16% Emulgator 2 |
| 3% Emulgator 2 | 5% Emulgator 3 |
| 5% Luviskol VA 64 | 5% Ultrasil VN 3 |
| | 1% wasserfreie Zitronensäure |
| Rest: n-Methylpyrrolidon | Rest: Sillitin Z |

| | |
|---|---|
| Emulgator 1: | Dodecylbenzolsulphonsäuremonoethanolaminsalz |
| Emulgator 2: | Nonylphenol-10-glykolether |
| Emulgator 3: | Alkylarylpolyglykolether |
| Ultrasil VN 3 ≙ | hochdisperses amorphes Siliziumdioxid |
| Sillitin Z ≙ | gemahlene Kieselsäure |
| Luviskol VA 64 ≙ | Vinylpyrrolidon-Vinylacetat-(60/40)-Copolymerisat |

## Auswertung der biologischen Wirksamkeit

Zur Prüfung der Wirksamkeit des Wirkstoffes in der erfindungsgemäßen Formulierung, werden junge Tomatenpflanzen mit wäßrigen Zubereitungen der Formulierung mit unterschiedlichen Konzentrationen bis zur Tropfnässe bespritzt. Zum Vergleich werden weitere Tomatenpflanzen mit wäßrigen Zubereitungen einer Standardformulierung in vergleichbaren Konzentrationen des Wirkstoffes separat behandelt. Nach Antrocknen des Spritzbelages werden sämtliche Pflanzen mit wäßrigen Sporensuspensionen des Pilzes Phytophtera infestans inokuliert. Die Pflanzen werden in einer Inkubationskabine bei 20 °C und 100% relativer Feuchtigkeit aufgestellt. Die Auswertung des Pflanzenbefalls in Abhängigkeit von der Konzentration des Wirkstoffes wird 3 Tage nach der Inokulation durchgeführt.

Bei diesem Test setzt die Wirkung der erfindungsgemäßen Formulierung bei Wirkstoffkonzentrationen, die um einen Faktor von 2 kleiner als die der Standardformulierung sind, ein.

## Beispiel 10

Der Wirkstoff 4-Chlor-N,3'-bis(4-chlorphenyl)-4',5'-dihydro-(f,4'-bi-fH-pyrazol)-f'-carboxamid wird als erfindungsgemäßes organisches Konzentrat unter Verwendung der in der folgenden Tabelle mengenmäßig aufgeführten Bestandteile formuliert. Nach Verdünnung des Konzentrates mit Wasser wird eine lichtdurchlässige wäßrige kolloidale Dispersion erhalten, die gegen Kristallisation und Sedimentieren beständig ist. Wird das Polymerisat Luvisol VA 30® weggelassen oder durch ein reines Polyvinylpyrrolidon wie Luvisol K 30® ersetzt, werden nach Verdünnung des Konzentrates mit Wasser trübe Dispersionen mit groben Teilchen erhalten, die sich innerhalb weniger Minuten absetzen. Zum Vergleich der biologischen Wirksamkeit wird eine Standardformulierung des Wirkstoffkonzentrates wie in der Tabelle angegeben zubereitet.

| erfindungsgemäße Formulierung | | Standardformulierung 1: | SC 200 |
|---|---|---|---|
| Lösungsmittel: | 54,0% N-methylpyrrolidon | Lösungsmittel: | 20,0% BP<br>Enerthen 2367<br>10,0% Propandiol |
| Emulgator: | 13,0% Emulgator 1 | Emulgator: | 5.0% Emulgator PS 29<br>0,5% Renex 36 |
| Polymerisat:<br>Wirkstoffanteil: | 13,0% Luviskol VA 64<br>20,0% | Verdickungsmittel: | 0,1% Kalzan S |
| | | Microbizid:<br>Wirkstoffanteil: | 0.1% Preventol D2<br>19% |
| | | Rest: | entsalztes Wasser |

**Auswertung der biologischen Wirksamkeit**

**a) Phaedon cochleariae**

Zur Prüfung der Wirksamkeit des Wirkstoffs in der erfindungsgemäßen Formulierung gegen Phaedon cochleariae werden Kohlblätter in wäßrige Zubereitungen der Formulierung mit unterschiedlichen Konzentrationen getaucht. Zum Vergleich werden weitere Kohlblätter mit wäßrigen Zubereitungen der Standardformulierung in vergleichbaren Konzentrationen des Wirkstoffes separat behandelt. Nach dieser Behandlung werden Larven des Meerrettichblattkäfers (Phaedon cochleariae) auf die Blätter aufgelegt. Nach 7 Tagen wird die Sterblichkeitsrate der Larven berechnet. Als Maß für die relative biologische Wirksamkeit der erfindungsgemäßen Formulierung im Vergleich zu der Standardformulierung bestimmt man das Verhältnis der Konzentrationen bei einer Sterblichkeitsrate von 50% nach folgender Gleichung: relative Wirksamkeit = LD 50 (erfindungsgemäße Formulierung)/LD 50 (Standardformulierung). Bei diesem Test zeigt die erfindungsgemäße Formulierung eine mehr als 8 Mal höhere Wirksamkeit.

**b) Plutella xylostella**

Zur Prüfung der Wirksamkeit der erfindungsgemäßen Formulierung gegen Plutella xylostella werden Kohlblätter (Brassica oleracea) in wäßrige Zubereitungen der Formulierung mit unterschiedlichen Konzentrationen getaucht. Zum Vergleich werden weitere Blätter mit wäßrigen Zubereitungen der Standardformulierung in vergleichbaren Konzentrationen des Wirkstoffes separat behandelt. Nach der Behandlung werden Raupen der Kohlschabe (Plutella xylostella) auf die Blätter aufgelegt. Nach 7 Tagen wird die Sterblichkeitsrate der Raupen gemäß dem in Beispiel 10a beschriebenen Verfahren berechnet. Bei diesem Test beträgt die relative Wirksamkeit der erfindungsgemäßen Formulierung 30.

**c) Sporoptera frugiperda**

Zur Prüfung der Wirksamkeit der erfindungsgemäßen Formulierung gegen Sporoptera frugiperda werden Kohlblätter (Brassica oleracea) in wäßrige Zubereitungen der Formulierung mit unterschiedlichen Konzentrationen getaucht. Zum Vergleich werden weitere Blätter mit wäßrigen Zubereitungen einer Standardformulierung in vergleichbaren Konzentrationen des Wirkstoffes separat behandelt. Nach der Behandlung werden Raupen des Eulenfalters (Poroptera frugiperda) auf die Blätter aufgelegt. Nach 7 Tagen wird die Sterblichkeitsrate der Raupen gemäß des in Beispiel 10a beschriebenen Verfahrens bewertet. Bei diesem Versuch beträgt die relative Wirksamkeit der erfindungsgemäßen Formulierung 20.

**d) Heliotis viriscens**

Zur Prüfung der Wirksamkeit der erfindungsgemäßen Formulierung gegen Heliotis viriscens werden Kohlblätter (Brassica oleracea) in wäßrige Zubereitungen der Formulierung mit unterschiedlichen Konzentrationen getaucht. Zum Vergleich werden weitere Blätter mit wäßrigen Zubereitungen einer Standardformulierung in vergleichbaren Konzentrationen des Wirkstoffes behandelt. Nach der Behandlung werden Tabakknospenraupen (Heliotis viriscens) auf die Blätter aufgelegt. Nach 7 Tagen wird die Sterblichkeitsrate der

Knospenraupen gemäß dem in Beispiel 10a beschriebenen Verfahren berechnet.

Bei diesem Versuch beträgt die relative Wirksamkeit der erfindungsgemäßen Formulierung 7,5.

**Patentansprüche**

**1.** Kolloidal dispergierbare flüssige Wirkstoff-Formulierung, enthaltend
als Komponente A einen schwerlöslichen Wirkstoff, als Komponente B ein Copolymerisat aus Vinylpyrrolidon und/oder Vinylcaprolactam und mindestens einem hydrophoben Comonomeren, das die Wasserlöslichkeit des Polymeren herabsetzt, als Komponente C ein oder mehrere Tenside, gegebenenfalls weitere Zusatzstoffe und/oder ein mit den Komponenten A und B bzw. D mischbare Verdünnungsmittel als Komponenten D bzw. E.

**2.** Dispergierbare flüssige Wirkstoff-Formulierung, gemäß Anspruch 1, enthaltend
als Komponente B ein statistisches Copolymerisat aus Vinylpyrrolidon und einem Vinylester der Formel

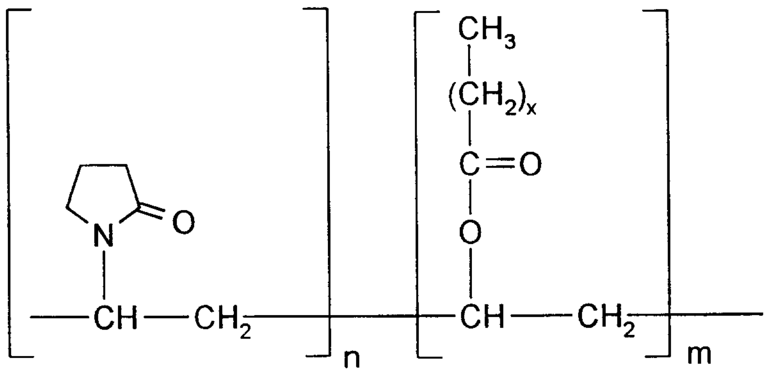

,

in welcher
X eine Zahl 0 bis 16, und n und m größer 1 sind.

**3.** Zubereitung gemäß Anspruch 1, dadurch gekennzeichnet, daß der Gewichtsanteil des hydrophoben Comonomeren am Polymer 5 bis 90 % beträgt.

**4.** Zubereitungen gemäß Anspruch 1, dadurch gekennzeichnet, daß die Komponente A ein Wirkstoff aus der Gruppe Dihydropyridine, Azole, Nitromethylen, Arylpyrazoline, Valinamidcarbamate, Benzthiophencarboxamide und/oder Benzoylharnstoffe darstellt.

**5.** Verwendung der Wirkstoff-Formulierungen nach Anspruch 1 in Form von Konzentraten oder nach Verdünnung mit Wasser als Arzneimittel oder Pflanzenschutzmittel.

**6.** Wäßrige Pflanzenschutzmittel enthaltend eine Wirkstoff-Formulierung nach Anspruch 1.

**7.** Wäßrige Arzneimittel enthaltend eine Wirkstoff-Formulierung nach Anspruch 1.

**8.** Verfahren zur Herstellung von wäßrigen Formulierungen schwerlöslicher Wirkstoffe, dadurch gekennzeichnet, daß man den Wirkstoff (A) mit einem Copolymerisat (B) aus Vinylpyrrolidon und einem hydrophoben Comonomeren und einem Tensid (C) in Wasser mischt.